# EUROPÄISCHE PATENTSCHRIFT

(11) **EP 2 666 445 B1**
(45) Veröffentlichungstag und Bekanntmachung des Hinweises auf die Patenterteilung: **22.07.2015**
(21) Anmeldenummer: 13167163.8
(22) Anmeldetag: 09.05.2013
(51) Int. Cl.: A61F 5/01

(54) **Sprunggelenkorthese**
Ankle joint orthesis
Orthèse de l'articulation de la cheville

(30) Priorität: 24.05.2012 DE 102012010240
(43) Veröffentlichungstag der Anmeldung: 27.11.2013
(73) Patentinhaber: medi GmbH & Co. KG, 95448 Bayreuth (DE)
(72) Erfinder: Bauer, Patrick, 91275 Auerbach (DE); Kübrich, Wolfgang, 96215 Lichtenfels (DE); Hoffeins, Peter, 95448 Bayreuth (DE)
(74) Vertreter: Blaumeier, Jörg

(56) Entgegenhaltungen:
- WO-A1-00/01328
- WO-A1-01/60289
- DE-C2- 4 291 109
- US-A- 5 833 640
- US-A1- 2011 144 554

## Beschreibung

Die Erfindung betrifft eine Sprunggelenkorthese, ausgebildet zur Immobilisierung des Sprunggelenks.

Verletzungen des Sprunggelenks ereignen sich täglich zuhauf. Zur konservativen Behandlung wird häufig eine Sprunggelenkorthese verwendet, die der Stabilisierung respektive Immobilisierung des Fußes dient, derart, dass der Fuß weder seitlich knicken kann, noch nach oben und unten gebeugt werden kann. Bekannte Orthesen bestehen üblicherweise aus einem textilen Flächengebilde, das in seiner Form der Anatomie des Beins und Fußes im Bereich des Sprunggelenks angepasst gestrickt ist. Dieses textile Flächengebilde umfasst in der Tragstellung das Sprunggelenk und die angrenzenden Bein- und Fußbereiche allseitig. Um es anzulegen kann es geweitet oder geöffnet werden, so dass der Patient quasi wie in einen Schuh einsteigen kann. Anschließend wird das textile Flächengebilde über geeignete Spannmittel, üblicherweise einen Schnürsenkel, der entsprechend geführt und gezogen wird, zusammengezogen, so dass sich das textile Flächengebilde fest um den Sprunggelenksbereich legt. Durch entsprechend festes Zuziehen ergibt sich einerseits ein stabiler Umgriff, darüber hinaus wird hierüber auch eine eventuelle Schwellung komprimiert.

Bekannt ist es ferner, zur weiteren Stabilisierung hinsichtlich eines seitlichen Umknickens ein oder mehrere Stabilisierungselemente außenseitig an das textile Flächengebilde anzulegen und mittels eines oder mehrerer Gurte, die in vorgegebener, definierter Zügelung um das textile Flächengebilde und das oder die Stabilisierungselemente zu schlingen sind, zu fixieren. Zwar kann hierüber eine noch bessere Seitenstabilisierung erreicht werden, jedoch ist das Ansetzen des oder der Stabilisierungselemente wie auch deren Fixierung respektive das Zügeln des oder der Gurte aufwendig und insbesondere für Personen, die beispielsweise alters- oder verletzungsbedingt Schwierigkeiten haben, in den Fußgelenksbereich zu gelangen, schwierig.

Eine Sprunggelenkorthese gemäss dem Oberbegriff von Anspruch 1 ist aus der WO 00/01328 (Fig. 11) bekannt.

Der Erfindung liegt damit das Problem zugrunde, eine Sprunggelenkorthese anzugeben, die einerseits eine sehr gute Seitenstabilisierung aufweist, andererseits aber einfach konfiguriert ist, so dass sie einfach und ohne großen Aufwand angelegt werden kann.

Zur Lösung dieses Problems ist bei einer Sprunggelenksorthese erfindungsgemäß ein Gestrickteil, das in seiner Form der Anatomie des Beins und Fußes im Bereich des Sprunggelenks angepasst gestrickt ist, und das in der Tragstellung das Sprunggelenk und die angrenzenden Bein- und Fußbereiche allseitig umfasst, und das zum Anlegen abschnittsweise geöffnet oder geweitet werden kann und in der Tragstellung über Spannmittel zusammengezogen und gespannt werden kann, sowie des Weiteren ein einteiliges Stabilisierungselement mit einem in der Tragstellung die Ferse aufnehmenden Fersenabschnitt, einem daran anschließenden, seitlich am Sprunggelenk vorbeilaufenden Seitenabschnitt und einem daran anschließenden, die Wade rückseitig umgreifenden Wadenabschnitt vorgesehen, wobei an der Außenseite des Stabilisierungselements ein oder mehrere erste Verbindungselemente und an der Innenseite des Gestrickteils ein oder mehrere zweite Verbindungselemente vorgesehen sind, derart, dass das in das Gestrickteil einsetzbare Stabilisierungselement durch Verbinden des oder der ersten Verbindungselemente mit dem oder den zweiten Verbindungselementen im Gestrickteil lösbar fixierbar ist.

Die erfindungsgemäße Sprunggelenkorthese ist zweiteilig ausgeführt, bestehend aus dem Gestrickteil und dem Stabilisierungselement. Das Stabilisierungselement, das bevorzugt ein Kunststoffbauteil ist, weist eine definierte Form auf, bestehend aus drei ausgezeichneten Abschnitten, nämlich zum einen einem Fersenabschnitt, der in der Tragstellung unterhalb der Ferse angeordnet ist und die Ferse seitlich umgreift, einem an dem Fersenabschnitt anschließenden Seitenabschnitt, der längs des Sprunggelenks die zur Wade hochläuft, sowie einem an den Seitenabschnitt anschließenden Wadenabschnitt, der quasi halbschalenartig die Wade umgreift. Dieses Stabilisierungselement dient der Aussteifung und der noch besseren Verhinderung des seitlichen Knickens, als dies bereits durch das Gestrickteil möglich ist.

Dieses Stabilisierungselement wird nun nicht außenseitig an das Gestrickteil angelegt, wo es sodann, siehe obige Beschreibung, in umständlicher und aufwendiger Weise zu fixieren ist. Vielmehr wird erfindungsgemäß dieses Stabilisierungselement in das anatomisch bereits vorgeformte Gestrickteil eingesetzt und dort über am Stabilisierungselement sowie am Gestrickteil vorgesehene Verbindungselemente lösbar fixiert. Dies ermöglicht es, vor dem Anlegen das Stabilisierungselement bereits in das Gestrickteil einzusetzen, wobei die korrekte Positionierung des Gestrickteils sehr einfach ist, da die ersten und zweiten Verbindungselemente an entsprechenden Positionen angeordnet sind, die so gewählt sind, dass das Stabilisierungselement in der definierten Verbindungsstellung automatisch ideal im Gestrickteil positioniert ist. In einer anderen Stellung kann das Stabilisierungselement nicht im Gestrickteil fixiert werden. Hat der Patient nun das Stabilisierungselement in das Gestrickteil eingesetzt, so kann er ohne Weiteres in die Orthese einsteigen und mit dem Fuß einerseits in das Stabilisierungselement schlüpfen, andererseits in das Gestrickteil. Nach Anlegen der Orthese ist lediglich das Gestrickteil über das Spannmittel zuzuziehen, also der Schnürsenkel oder dergleichen festzuschnüren. Ist dies erfolgt, ist die Sprunggelenkorthese fertig angelegt, der Fuß ist, nachdem das Stabilisierungselement in korrekter und optimaler Position im Gestrickteil vor dem Anlegen bereits positioniert wurde, folglich ebenfalls in optimaler Weise sowohl im Stabilisierungselement als auch im Gestrickteil aufgenommen. Irgendwelche aufwendigen sonstigen Handhabungen im Hinblick auf die Fixierung des Stabilisierungselements, wie sie im Stand der Technik erforderlich sind, sind bei der erfindungsgemäßen Orthese gerade nicht mehr erforderlich. Auch ist die Art des Anlegens der Sprunggelenkorthese quasi selbsterklärend, da es wie gesagt lediglich erforderlich ist, das Stabilisierungselement über die Verbindungselemente im Gestrickteil anzuordnen, wonach lediglich die Orthese anzulegen und zu schnüren ist. Irgendwelche komplizierten Zügelwege eines Gurtes oder dergleichen sind hier gerade nicht zu lernen.

Als erste und zweite Verbindungselemente, über die das Stabilisierungselement und das Gestrickteil lösbar verbunden werden, sind bevorzugt Klettverbinder vorgesehen. Diese können am Stabilisierungselement beispielsweise an der Außenseite des Wadenabschnitts, vorzugsweise mittig, und an der Außenseite des Fersenabschnitts, vorzugsweise mittig, vorgesehen sein. An entsprechenden, in der Tragstellung gegenüberliegenden Positionen des Gestrickteils finden sich die entsprechenden zweiten Klettverbinder. Alternativ können natürlich auch Druckknopfverbinder oder Ähnliches vorgesehen sein.

Wie beschrieben ist das Stabilisierungselement vorzugsweise aus Kunststoff gefertigt. Da es lösbar ist, kann es zu Reinigungszwecken sehr einfach dem Gestrickteil entnommen werden. Als Kunststoffbauteil ist es auch ohne Weiteres reinigbar.

Die ersten Verbindungselemente am Stabilisierungselemente, insbesondere die Klettverbinder, können entweder auf die Außenseite des Stabilisierungselements aufgeklebt werden, denkbar ist es aber auch, sie unmittelbar in das aus Kunststoff gefertigte Stabilisierungselement einzuspritzen, also materialmäßig direkt mit dem Stabilisierungselement zu verbinden.

Gemäß einer besonders vorteilhaften Weiterbildung ist vorgesehen, dass das Stabilisierungselement abschnittsweise unterschiedliche Steifigkeiten aufweist, derart, dass der Seitenabschnitt härter ist als zumindest Teile des Wadenabschnitts und des Fersenabschnitts. Zentrale Funktion des Stabilisierungselements ist die Seitenstabilisierung, die ein seitliches Umknicken verhindert. Zu diesem Zweck ist es vorteilhaft, den Seitenabschnitt relativ hart auszuführen. Für einen guten Tragekomfort ist es jedoch vorteilhaft, insbesondere die Bereiche, die beim Tragen mitunter belastet werden bzw. die in feste Anlage an den Körper beim Schnüren gebracht werden, nämlich Bereiche des Fersenabschnitts wie auch des Wadenabschnitts, relativ weich zu gestalten, so dass sie flexibel und anpassbar sind, sich also der Form des Fuß- und Wadenbereichs gut anpassen können. Dies wird erreicht, indem das aus Kunststoff gefertigte Stabilisierungselement gerade in diesen sensiblen Bereichen weicher eingestellt ist als im Seitenabschnitt. Dabei kann auch der Fersenabschnitt in Verlängerung des Seitenabschnitts gleich oder ähnlich hart sein wie der Seitenabschnitt, d. h., dass der harte Seitenabschnitt quasi noch etwas bis in den Fersenabschnitt, vorzugsweise bis unter die Ferse des Patienten, gezogen ist, während die anderen Fersenabschnittsbereiche, insbesondere diejenigen, die die Ferse seitlich umfassen, weich eingestellt sind. Die Ausgestaltung des Stabilisierungselements aus Kunststoff lässt dabei eine beliebige Anpassung respektive Weichheitseinstellung zu, indem entsprechend unterschiedliche Kunststoffe respektive Kunststoffe gleichen Typs, jedoch unterschiedlicher Shore-Härte zur Bildung des Stabilisierungselements verwendet werden. Die Herstellung ist auf einfache Weise durch Mehrkomponentenspritzen möglich.

Eine zweckmäßige Weiterbildung der Erfindung sieht vor, das Gestrickteil aus einem Gestrickabschnitt und einer Zunge zu bilden, die insbesondere über ein schalenförmiges Einlageteil härter als der Gestrickabschnitt ausgelegt ist, wobei die Zunge herausklappbar am Gestrickabschnitt befestigt ist. Das Gestrickteil besteht also aus einem hinreichend rigiden Gestrickabschnitt, der so gestrickt ist, dass er der Fußform im übergreifenden Bereich angepasst ist. Er ist im Bereich des Schienbeins respektive Oberfußes offen, hier wird der Gestrickabschnitt über das Stabilisierungselement zum Fixieren zugezogen. An dem Gestrickabschnitt ist eine Zunge befestigt, die ausgeklappt werden kann, so dass folglich das Gestrickteil weit geöffnet werden kann, was den Einstieg in die Orthese auch bei integriertem Stabilisierungselement sehr erleichtert. Das Zungenteil wird nach Einsteigen in die Orthese an das Bein angelegt, also in den Gestrickabschnitt zurückgeschwenkt, der Gestrickabschnitt umgreift mit seinen Längsseiten die Zunge, über die der Schnürsenkel geführt ist. Wird diese nun zugezogen, so werden die Gestrickabschnittränder fest zusammengezogen und über die Zunge gezogen.

Dabei ist es aus Stabilisierungsgründen besonders vorteilhaft, wenn die Zunge hart ausgelegt ist, was mittels eines schalenförmigen Einlageteils ohne Weiteres möglich ist. Selbstverständlich ist eine entsprechende Polsterung an der Zungeninnenseite vorgesehen. Das Gestrickteil selbst ist ein rigides Gestrick, das möglichst nahtlos anatomisch angepasst gestrickt ist. Die Zugfestigkeit und Rigidität des Gestricks wird vorzugsweise durch Köperbindungen und eine Schusseinlage erreicht, die Elastizität im Fersenbereich, die erforderlich ist, damit sich das Gestrickteil in diesem Bereich der Fersenform anpassen kann, wird vorzugsweise durch "Rechts-Rechts"-Bindung mit partieller Schusseinlage erzielt, ausgeführt vorzugsweise mit elastischen Strick- und Schussfadenmaterialien. Andere Bindungstechniken, Strick- und Schussfadenmaterialien sind denkbar und hinlänglich bekannt. Die Form des Gestricks wird komplett bevorzugt durch die sogenannte Spickeltechnik erreicht, eine weitere Konfektion und Nahtbildung zur Formgebung ist bei Anwendung dieser Technik nicht von Nöten.

Als Spannmittel ist wie beschrieben vorzugsweise ein Schnürsenkel vorgesehen, mit dem der Gestrickabschnitt über der Zunge zusammengezogen werden kann. Hierfür sind zweckmäßigerweise an den Rändern des Gestrickabschnitts im fussseitigen Bereich Ghilly-Ösen und im beinseitigen Bereich Haken vorgesehen, durch bzw. um die der Schnürsenkel zum Spannen geführt ist. Der Schnürsenkel kann ein integriertes Klemmsystem besitzen, das es ermöglicht, den Schnürsenkel nach Zuziehen einfach zu verklemmen, ohne eine Schlaufe binden zu müssen.

Die direkte Integration des Stabilisierungselements, das wie ausgeführt direkt hautseitig am Bein respektive Gelenksbereich anliegt, in Verbindung mit der außenseitigen Fixierung und Verspannung über das Gestrickteil nebst Zunge führt bereits zu einer hervorragenden Immobilisierung des Sprunggelenks. Denn infolge der innenseitigen Anordnung und folglich dem direkten Kontakt des Stabilisierungselements im zu stabilisierenden Bereich ist eine unmittelbare Stabilisierungswirkung erreichbar, anders als bei bisher bekannten Orthesen, bei denen die Stabilisierungswirkung des Stabilisierungselements nur indirekt erreicht wird, da zwischen Stabilisierungselement und dem zu stabilisierenden Bereich immer ein textiles Flächengebilde angeordnet ist. Dort ist also immer noch eine indirekte Stabilisierung erreichbar. Um bei der erfindungsgemäßen Orthese jedoch die bereits gegebene hervorragende Stabilisierung noch weiter zu verbessern, was üblicherweise nur in besonderen Problemfällen, die sich aus der Art der Verletzung ergeben, der Fall sein kann, kann erfindungsgemäß ferner ein Gurt vorgesehen sein, an dessen beiden Enden dritte Verbindungselemente vorgesehen sind, die mit am Gestrickteil außenseitig vorgesehenen vierten Verbindungselementen lösbar verbindbar sind, wobei der Gurt zur weitergehenden Immobilisierung in der Tragstellung um das Gestrickteil in einer 8er-Zügelung geführt ist. Über diesen Gurt, der ausschließlich der Immobilisierung, jedoch nicht der Fixierung eines Stabilisierungselements oder Ähnlichem dient, kann eine nahezu vollständige Immobilisierung des Fußgelenks erreicht werden. Sofern erforderlich kann also über den Gurt eine Aufrüstung der Orthese erfolgen, beispielsweise dann, wenn nach Anlegen der "Basisorthese" bestehend aus Stabilisierungselement und Gestrickteil eine Schwellung abgeschwollen ist, nachfolgend jedoch eine komplette Immobilisierung temporär erforderlich ist. Nach einer bestimmten Behandlungszeit kann dann der Gurt entfernt werden und die über die "Basisorthese" gegebene Stabilisierung ausreichend sein. Zum Ende der Behandlung hin kann sodann auch auf das Stabilisierungselement verzichtet werden, so dass das Sprunggelenk lediglich noch über das Gestrickteil fixiert ist.

Wie beschrieben sind am Gurt an seinen Enden die dritten Verbindungselemente vorgesehen, am Gestrickteil sind die vierten Verbindungselemente an den Seitenbereichen vorgesehen, zu denen die Gurtenden über die 8er-Zügelung geführt werden. Denkbar ist es aber auch zur Fixierung des Gurts vor dem eigentlichen Zügeln, in der Gurtmitte und an der Unterseite des Fersenbereichs des Gestrickteils ein weiteres drittes und viertes Verbindungselement vorzusehen.

Auch diese Verbindungselemente sind bevorzugt als Klettverbinder ausgeführt, denkbar sind aber auch Druckknopfverbindungen oder Ähnliches.

Weitere Vorteile, Merkmale und Einzelheiten der Erfindung ergeben sich aus dem im Folgenden beschriebenen Ausführungsbeispiel sowie anhand der Zeichnung. Dabei zeigen:
- Fig. 1: eine Perspektivansicht eines Gestrickteils einer erfindungsgemäßen Sprunggelenkorthese,
- Fig. 2: eine Perspektivansicht eines Stabilisierungselements der erfindungsgemäßen Orthese,
- Fig. 3: das Stabilisierungselement aus Fig. 2 in einer rückseitigen Ansicht,
- Fig. 4: die erfindungsgemäße Sprungorthese mit in das Gestrickteil eingesetztem Stabilisierungselement, und
- Fig. 5: die Sprunggelenkorthese aus Fig. 4 mit zusätzlich angebrachtem Gurt.

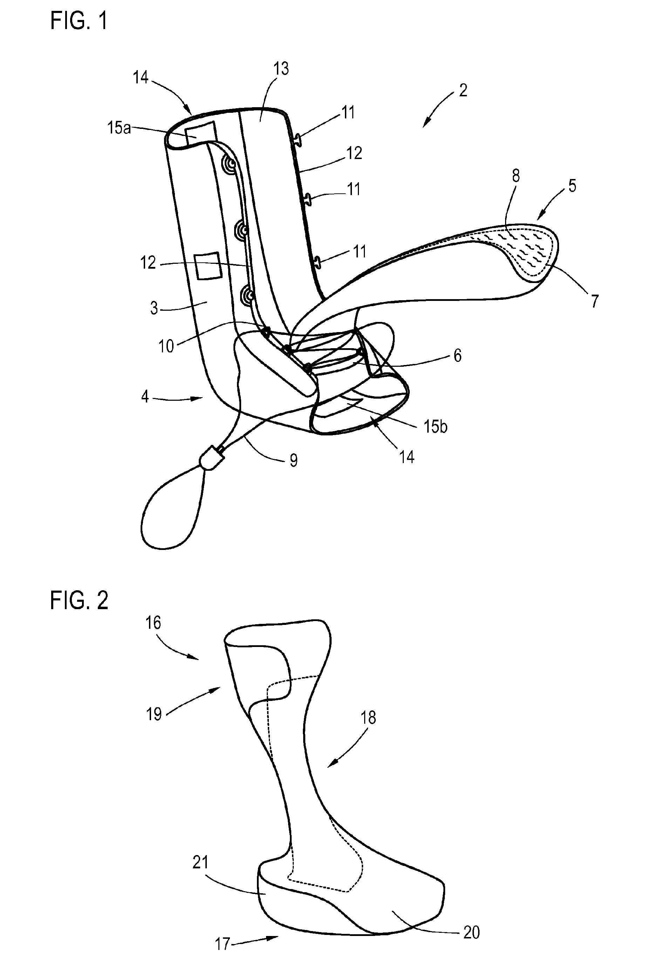

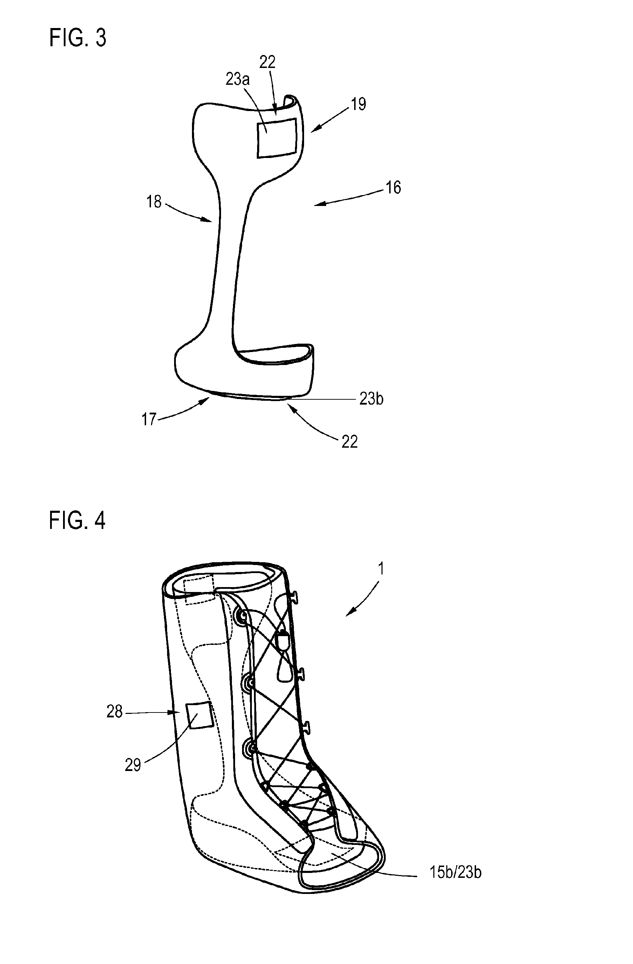

Fig.1 zeigt als Teil einer erfindungsgemäßen Sprunggelenkorthese 1, wie sie in Fig. 4 gezeigt ist, ein Gestrickteil 2, bestehend aus einem Gestrickabschnitt 3, der als nahtloses, rigides Gestrick derart gestrickt ist, dass er der Anatomie des Fußes angepasst ist. Seine Zugfestigkeit und seine Rigidität wird mittels Köperbindungen und einer Schusseinlage erreicht. Im Fersenbereich 4 ist er elastisch gestrickt, was vorzugsweise durch "Rechts-Rechts"-Bindung mit partieller Schusseinlage erzielt wird. Verwendet werden vorzugsweise elastische Strick- und Schussfadenmaterialien. Die anatomisch angepasste Form des Gestricks wird vorzugsweise durch die sogenannte Spickeltechnik erreicht.

Am Gestrickabschnitt 3 ist eine Zunge 5 beweglich angeordnet, wozu entsprechende elastische Gummizugverbindungen 6 vorgesehen sind, die am Gestrickteil 3 wie auch an der Zunge 5 befestigt, vorzugsweise angenäht sind. Die Zunge 5 ist also unlösbar mit dem Gestrickabschnitt 3 verbunden. Sie weist eine hier nur gestrichelt gezeigte, hinreichend stabile und halbschalenförmig ausgelegte Einlage 7 auf, die der Aussteifung der Zunge dient. Die Zunge ist also in sich gesehen sehr stabil, sie wird über die Einlage 7, beispielsweise eine geeignete Kunststoffschale, hinreichend ausgesteift. An der Innenseite der Zunge 5 ist eine Polsterung 8 vorgesehen, um eine gepolsterte Auflage am Bein des Patienten zu ermöglichen.

Vorgesehen ist ferner ein Spannmittel hier in Form eines Schnürsenkels 9, der durch eine Reihe von Ghilly-Ösen 10 geführt ist, so dass er nicht allzu leicht ausgefädelt werden kann. Zum endgültigen Spannen und Schnüren wird er sodann um mehrere Haken 11, die an den Rändern 12 des Gestrickteils 3 befestigt sind (wie auch die Ghilly-Ösen) geführt, wonach er zum Fixieren zugezogen respektive geschnürt wird.

An der Innenseite 13 des Gestrickabschnitts 3 sind im gezeigten Beispiel mehrere erste Verbindungselemente 14 an unterschiedlichen Positionen fest angeordnet. Bei diesen ersten Verbindungselementen handelt es sich vorzugsweise um Klettverbinder. Ein erster Klettverbinder 15a befindet sich im oberen Wadenbereich, ein zweiter Klettverbinder 15b befindet sich im Fersen- oder Fußbereich des Gestrickabschnitts 3. Diese Klettverbinder 15a, 15b dienen der Festlegung respektive Fixierung eines Stabilisierungselements, worauf nachfolgend noch eingegangen wird. Die Klettverbinder sind mit dem Gestrickabschnitt 3 fest vernäht oder verklebt. Alternativ zur Verwendung von Klettverbindern wären grundsätzlich auch Druckknöpfe oder Ähnliches verwendbar, jedoch sind insbesondere Klettverbinder besonders einfach zu befestigen und auch zu verbinden.

Die Fig. 2 und 3 zeigen ein Stabilisierungselement 16, das als Kunststoffbauteil ausgeführt ist. Es besteht aus drei Abschnitten, nämlich dem Fersenabschnitt 17, dem Seitenabschnitt 18 und dem Wadenabschnitt 19. Der Fersenabschnitt 17 ist schalenförmig ausgeführt, er weist einen unteren Fersenauflagebereich 20 auf, der in einen Seitenbereich 21 übergeht, mit dem die Ferse seitlich und hinten umgriffen wird. Er geht einstückig in den Seitenabschnitt 18 über, der sich vom Fußabschnitt 17 nach oben erstreckt und in der Tragstellung seitlich entlang des geschädigten Sprunggelenks verläuft. Dieser Seitenabschnitt 18 geht wiederum einstück in den Wadenabschnitt 19 über, der nach Art einer Halbschale in der Tragstellung die Wade rückseitig umgreift.

Der Seitenabschnitt 18 ist härter eingestellt als der Wadenabschnitt 19 wie auch weitestgehend der Fersenabschnitt 17. Er ist also steifer, weist eine andere Shore-Härte als die anderen Abschnitte auf. Sein Zweck ist allein die Stabilisierung im Hinblick auf eine Vermeidung eines Knickens des Sprunggelenks, weshalb eine hohe Steifigkeit von Vorteil ist. Wie in Fig. 2 gestrichelt angedeutet ist, zieht sich der Bereich der erhöhten Steifigkeit jedoch noch etwas bis in den Fersenabschnitt 17 in den Bereich der Fersenauflage 20, mithin also unter die Fußsohle respektive Ferse. Demgegenüber sind alle anderen Abschnitte des Stabilisierungselements 16 wesentlich weicher und flexibler, so dass sie sich ohne Weiteres der Fußrespektive Wadenform anpassen und anschmiegen können, mithin also es nicht zu irgendwelchen Druck- oder Reibstellen aufgrund zu steifen Materials in diesen Bereichen kommt.

Wie der rückseitigen Ansicht gemäß Fig. 3 zu entnehmen ist, sind am Wadenabschnitt 19, am Seitenabschnitt 18 sowie am Fersenabschnitt 17 erste Verbindungselemente 22 vorgesehen, die kongruent zum Verbindungselementtyp des Gestrickabschnitts 3 ausgelegt sind. Nachdem dort bevorzugt Klettverbinder 15a, 15b verwendet werden, sind folglich die ersten Verbindungselemente 22 ebenfalls als Klettverbinder 23a, 23b ausgeführt. Sie sind an Positionen am Stabilisierungselement 16 angeordnet, die so gewählt sind, dass das Stabilisierungselement 16, wenn es in das Gestrickteil 2 eingesetzt ist, automatisch optimal im Gestrickteil positioniert ist, was über die Verbindung der Klettverbinder 15a/23a und 15b/23b erfolgt.

Die ersten Verbindungselemente 22, also die Klettverbinder 23a, 23b sind vorzugsweise direkt in das Kunststoffmaterial des Stabilisierungselements 16 eingespritzt, so dass sich eine materialschlüssige Verbindung ergibt. Denkbar ist es aber auch, sich auf einfache Weise aufzukleben.

Zum Anlegen der Sprunggelenkorthese 1 ist es lediglich erforderlich, das Stabilisierungselement 16 in das Gestrickteil 2 einzusetzen. Hierzu wird das Gestrickteil 2 auf einfache Weise geöffnet, indem die Zunge 5 herausgeklappt wird, wie in Fig. 1 exemplarisch dargestellt ist. Sodann wird das Stabilisierungselement 16 in das Gestrickteil 2 eingesetzt, und soweit verschoben respektive positioniert, bis die Klettverbinder 15a/23a und 15b/23b einander gegenüberliegen respektive miteinander verbunden werden können. Nach deren Verbindung ist das Stabilisierungselement 16 fest und positionsgenau im Gestrickteil 2 respektive im Gestrickabschnitt 3 fixiert.

Sodann steigt der Patient in die nach wie vor noch offene Orthese ein. Hierbei wird der Fuß automatisch in das Stabilisierungselement 16 eingeführt. Mit seiner Ferse sitzt der Fuß auf dem Fersenauflagebereich 20 respektive im Fersenabschnitt 17, das Sprunggelenk liegt unmittelbar benachbart zum Seitenabschnitt 18, während die Wade von dem Wadenabschnitt 19 umgriffen ist. Zum Fixieren und Immobilisieren ist es nun lediglich noch erforderlich, die Zunge 5 einzuschwenken, so dass die Polsterung 8 schienbein- respektive oberfussseitig aufliegt. Die Seitenränder 12 des Gestrickabschnitts 3 liegen außen auf der Zunge 5. Nun ist lediglich noch der Schnürsenkel 9 entsprechend zu führen und zuzuziehen respektive zu schnüren, so dass die Gestrickabschnittränder 12 fest zueinander zugezogen werden, womit automatisch das rigide Gestrick fest um den Gelenkbereich gezogen wird. Hierbei wird die Zunge 5 mit ihrer Polsterung 8 ebenfalls gegen den Gelenkbereich gedrückt. Infolge der Rigidität des Gestrickabschnitts 3 ist damit aber auch das Stabilisierungselement 16 relativ zum Gelenkbereich respektive Fuß festgelegt, der Fuß wird über das Stabilisierungselement 16 sicher gegen ein seitliches Knicken geschützt. Eine Bewegung nach oben und unten ist hierdurch jedoch, abhängig von der Ausführung der Zunge, nicht zwangsläufig ausgeschlossen.

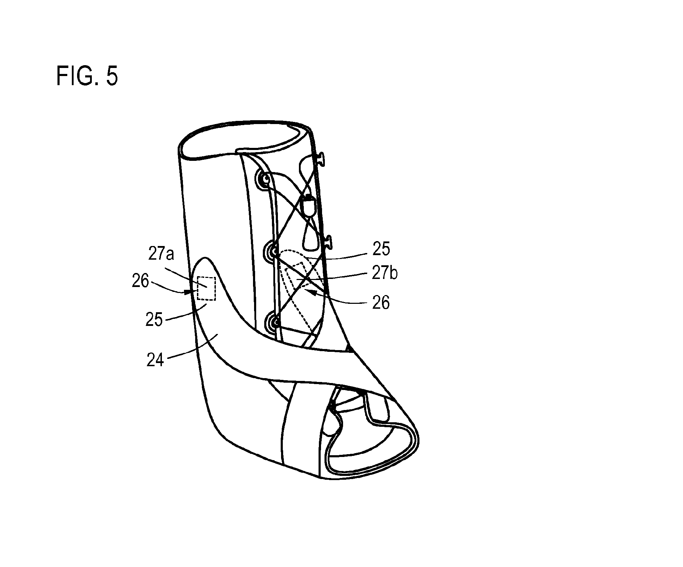

Um eine noch weitergehende Immobilisierung auch in Bezug auf diese Bewegung zu erreichen ist, siehe Fig. 5, ein Gurt 24 vorgesehen, an dessen beiden Enden 25 dritte Verbindungselemente 26, vorzugsweise wiederum in Form von Klettverbindern 27a, 27b vorgesehen. Am Gestrickabschnitt 3 sind außenseitig im Bereich der Seitenbereiche entsprechende vierte Verbindungselemente 28 vorgesehen (in Fig. 4 ist nur das eine gezeigt), die wiederum als Klettverbinder 29 ausgeführt sind. Auch in der Gurtmitte kann ein dritter Klettverbinder sein, entsprechend ist an der Unterseite des Gestrickabschnitts 3 ein vierter Klettverbinder, so dass der Gurt auch mittig fixiert werden kann.

Wie Fig. 5 zeigt, wird der Gurt 24 nun nach Art einer 8er-Zügelung geführt und um die bereits angelegte Sprunggelenkorthese 1 gezogen. Da der Gurt in sich stabil ist, mithin also sich nicht längt, kann durch entsprechendes straffes Anziehen eine weitergehende Immobilisierung erreicht werden.

Ersichtlich ist das maximal 3-teilige System insoweit völlig flexibel aufgebaut, als je nach Verletzungs- respektive Heilungszustand entweder alle drei Elemente, nur das Gestrickteil 2 und das Stabilisierungselement 16, oder nur das Gestrickteil 2 angelegt werden kann. Beispielsweise kann unmittelbar nach Zuziehen einer Verletzung mit einer entsprechenden Schwellung nur das Gestrickteil 2 mit eingesetztem Stabilisierungselement 16 angelegt werden. Nach Abschwellen kann zusätzlich der Gurt 24 angelegt werden. Mit zunehmendem Heilungserfolg kann der Gurt 24 entfernt werden, so dass nur noch das Gestrickteil 2 und das Stabilisierungselement 16 die Immobilisierung erwirken. Mit weitergehendem Heilungserfolg kann sodann das Stabilisierungselement entnommen werden und die Stabilisierung nur noch über das Gestrickteil 2 erfolgen.

## Patentansprüche

1. Sprunggelenkorthese (1), ausgebildet zur Immobilisierung des Sprunggelenks, umfassend ein einteiliges Stabilisierungselement (16) mit einem in der Tragstellung die Ferse aufnehmenden Fersenabschnitt (17), einem daran anschließenden Seitenabschnitt (18) und einem daran anschließenden, die Wade rückseitig umgreifenden Wadenabschnitt (19), wobei an der Außenseite des Stabilisierungselements (16) ein oder mehrere erste Verbindungselemente (22) vorgesehen sind, **gekennzeichnet durch** ein Gestrickteil (2), das in seiner Form der Anatomie des Beins und Fußes im Bereich des Sprunggelenks angepasst gestrickt ist und in der Tragstellung das Sprunggelenk und die angrenzenden Bein- und Fußbereiche allseitig umfasst, und das zum Anlegen abschnittsweise geöffnet oder geweitet werden kann und in der Tragstellung über Spannmittel (9) zusammengezogen und gespannt werden kann, wobei an der Innenseite (13) des Gestrickteils (2) ein oder mehrere zweite Verbindungselemente (14) vorgesehen sind, derart, dass das in das Gestrickteil (2) einsetzbare Stabilisierungselement (16), dessen Seitenabschnitt seitlich am Sprunggelenk vorbeiläuft, **durch** Verbinden des oder der ersten Verbindungselemente (22) mit dem oder den zweiten Verbindungselementen (14) im Gestrickteil (2) lösbar fixierbar ist.

2. Sprunggelenkorthese nach Anspruch 1, **dadurch gekennzeichnet, dass** als erste und zweite Verbindungselemente (22, 14) Klett- oder Druckknopfverbinder (23a, 23b, 23c, 15a, 15b, 15c) vorgesehen sind.

3. Sprunggelenkorthese nach Anspruch 1 oder 2, **dadurch gekennzeichnet, dass** ein erstes Verbindungselement (22) am Wadenabschnitt (19) und ein zweites Verbindungselement (14) am Wadenbereich des Gestrickteils (2) vorgesehen ist.

4. Sprunggelenkorthese nach einem der vorangehenden Ansprüche, **dadurch gekennzeichnet, dass** ein erstes Verbindungselement (22) am Fersenabschnitt (17) und ein zweites Verbindungselement (14) am Fersenbereich des Gestrickteils (2) vorgesehen ist.

5. Sprunggelenkorthese nach einem der vorangehenden Ansprüche, **dadurch gekennzeichnet, dass** das Stabilisierungselement (16) aus Kunststoff ist.

6. Sprunggelenkorthese nach Anspruch 5, **dadurch gekennzeichnet, dass** das es abschnittsweise unterschiedliche Steifigkeiten aufweist, derart, dass der Seitenabschnitt (18) härter ist als zumindest Teile des Wadenabschnitts (19) und des Fersenabschnitts (17).

7. Sprunggelenkorthese nach Anspruch 6, **dadurch gekennzeichnet, dass** der Fersenabschnitt (17) in Verlängerung des Seitenabschnitts (18) gleichermaßen hart wie der Seitenabschnitt (18) ist, welcher harte Bereich sich bis in den unteren Fersenabschnittsbereich erstreckt, während die anderen Bereiche des Fersenabschnitts (17) weicher sind.

8. Sprunggelenkorthese nach einem der vorangehenden Ansprüche, **dadurch gekennzeichnet, dass** das Gestrickteil (2) aus einem Gestrickabschnitt (3) und einer Zunge (5) besteht, die insbesondere über ein schalenförmiges Einlageteil (7) härter als der Gestrickabschnitt (3) ausgelegt ist, wobei die Zunge (5) herausklappbar am Gestrickabschnitt (3) befestigt ist.

9. Sprunggelenkorthese nach Anspruch 8, **dadurch gekennzeichnet, dass** als Spannmittel ein Schnürsenkel (9) vorgesehen ist, mit dem der Gestrickabschnitt (3) über die Zunge (5) zusammengezogen werden kann.

10. Sprunggelenkorthese nach Anspruch 9, **dadurch gekennzeichnet, dass** an den Rändern (12) des Gestrickabschnitts (3) im fußseitigen Bereich Ghilly-Ösen (10) und im beinseitigen Bereich Hacken (11) vorgesehen sind, durch bzw. um die der Schnürsenkel (9) zum Spannen geführt ist.

11. Sprunggelenkorthese nach einem der Ansprüche 8 bis 10, **dadurch gekennzeichnet, dass** an der Innenseite der Zunge (5) eine Polsterung (8) vorgesehen ist.

12. Sprunggelenkorthese nach einem der vorangehenden Ansprüche, **dadurch gekennzeichnet, dass** ferner ein Gurt (24) vorgesehen ist, an dessen beiden Enden dritte Verbindungselemente (26) vorgesehen sind, die mit am Gestrickteil (2) außenseitig vorgesehenen vierten Verbindungselementen (28) lösbar verbindbar sind, wobei der Gurt (24) zur weitergehenden Immobilisierung in der Tragstellung um das Gestrickteil (2) in einer 8er-Zügelung geführt ist.

13. Sprunggelenkorthese nach Anspruch 12, **dadurch gekennzeichnet, dass** in der Gurtmitte und an der Unterseite des Fersenbereichs des Gestrickteils (2) ein weiteres drittes und viertes Verbindungselement (26, 28) vorgesehen ist.

14. Sprunggelenkorthese nach Anspruch 12 oder 13, **dadurch gekennzeichnet, dass** die dritten und vierten Verbindungselemente (26, 28) Klett- oder Druckknopfverbinder (27a, 27b, 29) sind.

## Claims

1. Ankle joint orthosis (1), designed to immobilize the ankle joint, comprising a one-piece stabilizing element (16) with a heel portion (17) receiving the heel in the position of use, a side portion (18) which adjoins the heel portion (17), and a calf portion (19) which adjoins the side portion (18) and engages around the rear of the calf, wherein one or more first connector elements (22) are provided on the outside of the stabilizing element (16), **characterized by** a knitted part (2) which is knitted in a shape adapted to the anatomy of the leg and foot in the area of the ankle joint and which, in the position of use, completely encloses the ankle joint and the adjoining areas of the leg and foot, and which can be opened or widened in some areas in order to fit it in place and, in the position of use, can be drawn together and tightened by tightening means (9), wherein one or more second connector elements (14) are provided on the inside (13) of the knitted part (2), in such a way that the stabilizing element (16), which is insertable into the knitted part (2), and the side portion of which extends along the side of the ankle joint, can be fixed releasably in the knitted part (2) by connection of the one or more first connector elements (22) to the one or more second connector elements (14).

2. Ankle joint orthosis according to Claim 1, **characterized in that** hook-and-loop or press-stud fasteners (23a, 23b, 23c, 15a, 15b, 15c) are provided as first and second connector elements (22, 14).

3. Ankle joint orthosis according to Claim 1 or 2, **characterized in that** a first connector element (22) is provided on the calf portion (19), and a second connector element (14) is provided on the calf area of the knitted part (2).

4. Ankle joint orthosis according to one of the preceding claims, **characterized in that** a first connector element (22) is provided on the heel portion (17), and a second connector element (14) is provided on the heel area of the knitted part (2).

5. Ankle joint orthosis according to one of the preceding claims, **characterized in that** the stabilizing element (16) is made of plastic.

6. Ankle joint orthosis according to Claim 5, **characterized in that** it has different degrees of stiffness in some sections, in such a way that the side portion (18) is harder than at least parts of the calf portion (19) and of the heel portion (17).

7. Ankle joint orthosis according to Claim 6, **characterized in that** the heel portion (17), in a continuation of the side portion (18), is equally as hard as the side portion (18), said hard area extending into the lower area of the heel portion, whereas the other areas of the heel portion (17) are softer.

8. Ankle joint orthosis according to one of the preceding claims, **characterized in that** the knitted part (2) is composed of a knitted portion (3) and of a tongue (5), which is made harder than the knitted portion (3) particularly by means of a shell-shaped insert part (7), wherein the tongue (5) is secured on the knitted portion (3) in such a way that it can be folded outward.

9. Ankle joint orthosis according to Claim 8, **characterized in that** the tightening means provided is in the form of a shoelace (9), with which the knitted portion (3) can be drawn together over the tongue (5).

10. Ankle joint orthosis according to Claim 9, **characterized in that**, at the edges (12) of the knitted portion (3), ghilly eyelets (10) are provided in the foot area and hooks (11) are provided in the leg area, and the shoelace (9) is guided through these eyelets and around these hooks in order to provide tightening.

11. Ankle joint orthosis according to one of Claims 8 to 10, **characterized in that** padding (8) is provided on the inner face of the tongue (5).

12. Ankle joint orthosis according to one of the preceding claims, **characterized in that** a strap (24) is furthermore provided, at the two ends of which there are third connector elements (26) which can be releasably connected to fourth connector elements (28) provided on the outside of the knitted part (2), wherein the strap (24) is guided around the knitted part (2) in a figure-of-eight configuration, so as to provide further immobilization in the position of use.

13. Ankle joint orthosis according to Claim 12, **characterized in that** a further third and fourth connector element (26, 28) is provided at the center of the strap and on the underside of the heel area of the knitted part (2).

14. Ankle joint orthosis according to Claim 12 or 13, **characterized in that** the third and fourth connector elements (26, 28) are hook-and-loop or press-stud fasteners (27a, 27b, 29).

## Revendications

1. Orthèse de l'articulation de la cheville (1), configurée de façon à immobiliser l'articulation de la cheville, comprenant un élément de stabilisation en une seule pièce (16) avec une partie talonnière (17) contenant le talon dans la position de soutien, avec une partie latérale (18) qui s'y raccorde et avec une partie molletière (19) qui s'y raccorde et qui entoure le mollet par l'arrière, dans laquelle il est prévu un ou plusieurs premier(s) élément(s) de liaison (22) sur le côté extérieur de l'élément de stabilisation (16), **caractérisée par** une pièce de tricot (2), qui est tricotée de façon adaptée par sa forme à l'anatomie de la jambe et du pied dans la région de l'articulation de la cheville et qui enveloppe de tous les côtés, dans la position de soutien, l'articulation de la cheville et les régions avoisinantes de la jambe et du pied, qui peut être ouverte ou élargie en partie pour sa mise en place et qui peut être refermée et serrée par des moyens de serrage (9) dans la position de soutien, dans laquelle il est prévu sur le côté intérieur (13) de la pièce de tricot (2) un ou plusieurs deuxième(s) élément(s) de liaison (14), de telle manière que l'élément de stabilisation (16) insérable dans la pièce de tricot (2), dont la partie latérale se place latéralement le long de l'articulation de la cheville, puisse être fixé de façon détachable dans la pièce de tricot (2) par liaison du ou des premier(s) élément(s) de liaison (22) avec le ou les deuxième(s) élément(s) de liaison (14).

2. Orthèse de l'articulation de la cheville selon la revendication 1, **caractérisée en ce qu'**il est prévu comme premiers et deuxièmes éléments de liaison (22, 14) des liens à bande velcro ou des liens à boutons-pression (23a, 23b, 23c, 15a, 15b, 15c).

3. Orthèse de l'articulation de la cheville selon la revendication 1 ou 2, **caractérisée en ce qu'**il est prévu un premier élément de liaison (22) sur la partie molletière (19) et un deuxième élément de liaison (14) sur la région molletière de la pièce de tricot (2).

4. Orthèse de l'articulation de la cheville selon l'une quelconque des revendications précédentes, **caractérisée en ce qu'**il est prévu un premier élément de liaison (22) sur la partie talonnière (17) et un deuxième élément de liaison (14) sur la région talonnière de la pièce de tricot (2).

5. Orthèse de l'articulation de la cheville selon l'une quelconque des revendications précédentes, **caractérisée en ce que** l'élément de stabilisation (16) est en matière plastique.

6. Orthèse de l'articulation de la cheville selon la revendication 5, **caractérisée en ce qu'**elle présente des raideurs localement différentes, de telle manière que la partie latérale (18) soit plus dure qu'au moins des régions de la partie molletière (19) et de la partie talonnière (17).

7. Orthèse de l'articulation de la cheville selon la revendication 6, **caractérisée en ce que**, dans le prolongement de la partie latérale (18), la partie talonnière (17) est aussi dure que la partie latérale (18), cette région dure s'étendant jusque dans la région inférieure de la partie talonnière, tandis que les autres régions de la partie talonnière (17) sont plus souples.

8. Orthèse de l'articulation de la cheville selon l'une quelconque des revendications précédentes, **caractérisée en ce que** la pièce de tricot (2) se compose d'une partie tricotée (3) et d'une patte (5), qui est de nature plus dure en particulier sur une partie d'insertion en forme de coquille (7) que la partie tricotée (3), la patte (5) étant fixée de façon rabattable à la partie tricotée (3).

9. Orthèse de l'articulation de la cheville selon la revendication 8, **caractérisée en ce qu'**il est prévu comme moyen de serrage un lacet (9), avec lequel la partie tricotée (3) peut être refermée sur la patte (5).

10. Orthèse de l'articulation de la cheville selon la revendication 9, **caractérisée en ce qu'**il est prévu sur les bords (12) de la partie tricotée (3), dans la région du côté du pied des oeillets Ghilly (10) et dans la région du côté de la jambe des crochets (11), à travers lesquels ou autour desquels le lacet (9) est guidé pour le serrage.

11. Orthèse de l'articulation de la cheville selon l'une quelconque des revendications 8 à 10, **caractérisée en ce qu'**il est prévu un rembourrage (8) sur le côté intérieur de la patte (5).

12. Orthèse de l'articulation de la cheville selon l'une quelconque des revendications précédentes, **caractérisée en ce qu'**il est en outre prévu une lanière (24), aux deux extrémités de laquelle il est prévu des troisièmes éléments de liaison (26), qui peuvent être assemblés de façon détachable à des quatrièmes éléments de liaison (28) prévus du côté extérieur sur la pièce de tricot (2), la lanière (24) étant conduite autour de la pièce de tricot (2) en un laçage en 8 pour renforcer l'immobilisation dans la position de soutien.

13. Orthèse de l'articulation de la cheville selon la revendication 12, **caractérisée en ce qu'**il est prévu en plus un troisième et un quatrième éléments de liaison (26, 28) au milieu de la lanière et sur le côté inférieur de la région talonnière de la pièce de tricot (2).

14. Orthèse de l'articulation de la cheville selon la revendication 12 ou 13, **caractérisée en ce que** les troisièmes et les quatrièmes éléments de liaison (26, 28) sont des liens à bande velcro ou des liens à boutons-pression (27a, 27b, 29).
